# EUROPÄISCHE PATENTANMELDUNG

(11) **EP 3 399 301 A1**
(43) Veröffentlichungstag der Anmeldung: **07.11.2018**
(21) Anmeldenummer: 17169234.6
(22) Anmeldetag: 03.05.2017
(51) Int. Cl.: G01N 21/31, G01N 33/28, G01N 21/47, G01J 3/02, G01J 3/28

(54) **SPEKTROSKOPISCHE ERMITTLUNG DES ZUSTANDES EINES BETRIEBSSTOFFES EINER MASCHINE MITHILFE EINES TRAGBAREN GERÄTS, Z.B. EINES SMARTPHONES**

(71) Anmelder: Siemens Aktiengesellschaft, 80333 München (DE)
(72) Erfinder: Dannewitz, Karsten, 44797 Bochum (DE)

(57) **Zusammenfassung**

Die Erfindung betrifft ein Verfahren zur Ermittlung eines Zustandes eines Betriebsstoffes (1) einer Maschine (2). Weiterhin betrifft die Erfindung ein Serviceverfahren für eine Maschine (2), bei deren Betrieb ein Betriebsstoff (1) verwendet wird. Ferner betrifft die Erfindung ein tragbares Gerät (5) und ein nichtflüchtiges computerlesbares Speichermedium (13) zur Ermittlung eines Zustandes eines Betriebsstoffes (1) einer Maschine (2). Um die Ermittlung des Zustandes des Betriebsstoffes (1) der Maschine (2) zu vereinfachen, wird unter anderem vorgeschlagen, dass das Verfahren zur Ermittlung des Zustandes des Betriebsstoffes (1) der Maschine (2) folgende Verfahrensschritte aufweist:
- Bereitstellen einer Probe (3) des zu analysierenden Betriebsstoffes (1),
- Beaufschlagen der bereitgestellten Probe (3) mit einer ersten elektromagnetischen Strahlung (4) mittels eines tragbaren Gerätes (5),
- Aufnehmen einer zweiten elektromagnetischen Strahlung (6), welche von der Probe (3) während der Beaufschlagung mit der ersten elektromagnetischen Strahlung (4) ausgesandt wird, mittels des tragbaren Gerätes (5),
- Ermitteln des Zustandes des Betriebsstoffes (1) anhand einer Analyse der zweiten elektromagnetischen Strahlung (6) unter Berücksichtigung der ersten elektromagnetischen Strahlung (4).

## Beschreibung

Die Erfindung betrifft ein Verfahren zur Ermittlung eines Zustandes eines Betriebsstoffes einer Maschine. Weiterhin betrifft die Erfindung ein Serviceverfahren für eine Maschine, bei deren Betrieb ein Betriebsstoff verwendet wird. Ferner betrifft die Erfindung ein tragbares Gerät und ein nichtflüchtiges computerlesbares Speichermedium zur Ermittlung eines Zustandes eines Betriebsstoffes einer Maschine.

Derartige Vorrichtungen bzw. derartige Verfahren kommen beispielsweise bei einer Maschine zum Einsatz, zu deren Betrieb ein Betriebsstoff verwendet wird, der bspw. einem Verschleiß, einer zunehmenden Verunreinigung oder einer Alterung unterworfen ist.

Eine erste Aufgabe der Erfindung ist es, die Ermittlung des Zustandes des Betriebsstoffes der Maschine zu vereinfachen. Eine zweite Aufgabe der Erfindung ist es, ein verbessertes Serviceverfahren für derartige Maschinen bereitzustellen.

Eine Lösung der ersten Aufgabe ergibt sich durch ein Verfahren zur Ermittlung eines Zustandes eines Betriebsstoffes einer Maschine, wobei das Verfahren die folgenden Verfahrensschritte aufweist:
- Bereitstellen einer Probe des zu analysierenden Betriebsstoffes,
- Beaufschlagen der bereitgestellten Probe mit einer ersten elektromagnetischen Strahlung mittels eines tragbaren Gerätes,
- Aufnehmen einer zweiten elektromagnetischen Strahlung, welche von der Probe während der Beaufschlagung mit der ersten elektromagnetischen Strahlung ausgesandt wird, mittels des tragbaren Gerätes,
- Ermitteln des Zustandes des Betriebsstoffes anhand einer Analyse der zweiten elektromagnetischen Strahlung unter Berücksichtigung der ersten elektromagnetischen Strahlung.

Vorzugsweise umfasst die Maschine ein Getriebe, eine elektrische Maschine, insbesondere einen Elektromotor, einen elektrischen Generator oder einen Transformator, oder eine Wärmekraftmaschine, insbesondere einen Verbrennungsmotor.

Der Betriebsstoff umfasst insbesondere Öl und/oder Wasser. Vorzugsweise wird der Betriebsstoff zur Kühlung und/oder zur Schmierung der Maschine verwendet.

Das vorgeschlagene Verfahren zur Ermittlung des Zustandes des Betriebsstoffes ist dank des Einsatzes des tragbaren Gerätes besonders leicht durchzuführen. Bei vielen Anwendungsfällen kann der Zustand des Betriebsstoffes dadurch zum einen vor Ort bei der Maschine und zum anderen schnell, insbesondere innerhalb weniger Minuten, ermittelt werden. Dadurch entfallen bisher erforderliche, oftmals lange Wartezeiten bei der Zustandsermittlung des Betriebsstoffes von bspw. einer Woche und mehr. Die langen Wartezeiten sind bisher in vielen Fällen erforderlich, da die zu untersuchende Probe über den Postweg an ein Labor gesendet werden muss, um dann im Labor untersucht zu werden, ehe das Labor die entsprechende Rückmeldung, ggf. wiederum über den Postweg, geben kann.

Somit ermöglicht das vorgeschlagene Verfahren die Ermittlung des Zustandes des Betriebsstoffes während eines Inspektionstermins vorzunehmen und vorzugsweise abzuschließen, was bspw. durch einen Servicetechniker oder durch den Maschinenbetreiber selbst erfolgen kann.

Vorzugsweise umfasst der ermittelte Zustand des Betriebsstoffes die Aussage, ob der Betriebsstoff weiter brauchbar ist oder ob der Betriebsstoff ersetzt werden muss.

Um das vorgeschlagene Verfahren zur Ermittlung des Zustands des Betriebsstoffes durchzuführen, kann die Probe des Betriebsmittels bspw. in einen flachen Behälter gegeben werden. Die Abmessungen des Behälters sind vorzugsweise derart gewählt, dass die darin befindliche Probe eine Oberfläche bildet, die größer als eine Emissionseinheit, insbesondere ein Bildschirm, zur Emission der ersten elektromagnetischen Strahlung durch das tragbare Gerät ist. Bspw. kann der flache Behälter zumindest so groß wie das Format DIN A5 sein, was 148 mm x 210 mm entspricht. Vorzugsweise ist die innere Oberfläche des Behälters weiß oder reflektierend ausgestaltet, wodurch eine besonders zuverlässige Ermittlung ermöglicht wird.

Insbesondere kann dabei vorgesehen sein, dass ein vorgegebenes Volumen des Betriebsstoffes in einen Behälter mit vorgegebenen Abmessungen eingegeben wird, um bei waagrechter Ausrichtung des Behälters recht präzise eine gewünschte Füllhöhe der Probe des Betriebsstoffes in dem Behälter zu erhalten.

Anhand der von der Probe zurückgeworfenen elektromagnetischen Strahlung kann dabei auf den Zustand des Betriebsstoffes zurückgeschlossen werden, bspw. indem die zweite elektromagnetische Strahlung Strahlungsanteile enthält, wie sie für Eisen- oder Stahlspäne oder -abrieb, typisch sind. Andere Hinweise für den Zustand des Betriebsstoffes umfassen bspw. die Farbe des Betriebsstoffes, da sich bspw. Maschinenöl mit zunehmender Einsatzdauer dunkler färbt. Enthält die untersuchte Probe eine Emulsion zweier Stoffe, bspw. eine Wasser-Öl-Emulsion, kann das tragbare Gerät das Vorliegen einer derartigen Emulsion feststellen und ggf. quantitative Aussagen, bspw. einer emulgierten Wassermenge, treffen, wodurch ebenfalls Rückschlüsse auf den Zustand des Betriebsstoffes gezogen werden können. Vielfältige weitere Kriterien sind denkbar, welche auf der Beaufschlagung der Probe mit der ersten elektromagnetischen Strahlung und der Aufnahme der zweiten elektromagnetischen Strahlung beruhen und mittels welchen der Zustand des Betriebsstoffes untersucht und beurteilt werden kann.

Die Vorteile des vorgeschlagenen Verfahrens zur Ermittlung des Zustandes des Betriebsstoffes werden im Folgenden anhand einer beispielhaften Untersuchung der Inhaltsstoffe des Maschinenöls einer Arbeitsmaschine, hier eines Getriebes, speziell eines Industrie-, Wind- oder Getriebemotorengetriebes erläutert. Das Maschinenöl als Betriebsstoff wird auf die verschiedenen, möglichen Inhaltsstoffe untersucht, die Hinweise auf den Zustand des Maschinenöls geben können. Folglich wird das Maschinenöl vorzugsweise im Wesentlichen auf Verschleißmetalle aus Verzahnungs- und Lagerabrieb, wie bspw. Eisen oder Stahl, auf Verunreinigungen, wie bspw. Silizium und auf Additive, wie bspw. Phosphor, Kalzium oder Schwefel, untersucht, um eine Aussage zum Zustand des Getriebeöls machen zu können und das Erfordernis eines Ölwechsels erkennen zu können.

Gemäß alternativen Methoden werden die Inhaltsstoffe des Öls durch eine Spektralanalyse des Emissionsspektrums des Öls nach Bestrahlung mit einer "weißen" Lichtquelle bestimmt, so dass eine Reflexionsspektroskopie vorgenommen wird. Die spektrale Analyse des Lichtes erfolgt mit Hilfe eines Prismen- oder Gitterspektrometers. Dies wird typischerweise in einem Analyselabor durchgeführt. Hierzu muss eine Ölprobe an dem Getriebe abgelassen und an das Analyselabor per Post versandt werden.

Demgegenüber ist es bei dem vorgeschlagenen Verfahren zur Ermittlung des Zustandes des Betriebsstoffes nicht erforderlich, eine Ölprobe an ein Analyselabor zu schicken, da die erforderliche Untersuchung des Betriebsstoffs insbesondere vor Ort mittels des tragbaren Gerätes vorgenommen wird. Dank des vorgeschlagenen Verfahrens können Kosten und vor allem Zeit eingespart werden, da es im Vergleich zu alternativen Methoden wesentlich einfacher gestaltet ist und damit dennoch verlässliche Ergebnisse erzielt werden können.

Eine Lösung der zweiten Aufgabe ergibt sich durch ein Serviceverfahren für eine Maschine, bei deren Betrieb ein Betriebsstoff verwendet wird, wobei das Serviceverfahren die folgenden Verfahrensschritte aufweist:
- Ermitteln des Zustandes des Betriebsstoffes der Maschine nach dem oben erläuterten Verfahren oder nach einer der nachfolgend erläuterten vorteilhaften Ausgestaltungen dieses Verfahrens,
- falls der ermittelte Zustand des Betriebsstoffes unzureichend ist:
   Vornehmen einer elektronischen Bestellung von neuem Betriebsstoff, um den bisher verwendeten Betriebsstoff mit dem unzureichenden Zustand zu ersetzen, und/oder
   Vornehmen einer elektronischen Bestellung eines Servicetermins, um den bisher verwendeten Betriebsstoff mit dem unzureichenden Zustand zu ersetzen.

Der Betriebsstoff ist dabei insbesondere als unzureichend anzusehen, wenn die Maschine unter Verwendung dieses Betriebsstoffes nicht mehr ordnungsgemäß weiterbetrieben werden kann. Dies ist bspw. dann der Fall, wenn der Betriebsstoff durch Verschleiß, einer zunehmenden Verunreinigung oder einer Alterung zu einer Beschädigung der Maschine führen würde und folglich ersetzt werden müsste. Denkbar ist auch, dass die Ermittlung des Zustandes des Betriebsstoffes ergibt, dass noch eine gewisse Restlaufzeit der Maschine mit diesem Betriebsstoff vertretbar ist, allerdings dennoch schon der Ersatz des noch verwendeten Betriebsstoffes durch neuen Betriebsstoff einzuplanen ist.

Für den Fall, dass der Zustand des Betriebsstoffes unzureichend ist, sieht das vorgeschlagene Serviceverfahren zum einen vor, dass eine elektronischen Bestellung von neuem Betriebsstoff vorgenommen wird, um den bisher verwendeten Betriebsstoff mit dem unzureichenden Zustand zu ersetzen. Dies kann insbesondere mithilfe des tragbaren Gerätes erfolgen, welches datentechnisch mit einem entsprechenden Dienstleister verbunden ist. Zusätzlich oder alternativ ist für den Fall, dass der Zustand des Betriebsstoffes unzureichend ist, gemäß dem vorgeschlagenen Serviceverfahren vorgesehen, dass eine elektronische Bestellung eines Servicetermins vorgenommen wird, um den bisher verwendeten Betriebsstoff mit dem unzureichenden Zustand zu ersetzen. Die elektronische Bestellung des Servicetermins wird dabei vorzugsweise anhand des tragbaren Gerätes vorgenommen, welches wiederum datentechnisch mit einem entsprechenden Dienstleister verbunden ist. Der genannte Dienstleister kann insbesondere der Hersteller oder Verkäufer der Maschine oder des Betriebsstoffes sein, der entsprechende Dienstleistungen für den Betreiber der Maschine anbietet.

Ferner ergibt sich eine weitere Lösung der ersten Aufgabe durch ein tragbares Gerät zur Ermittlung eines Zustandes eines Betriebsstoffes einer Maschine, wobei das tragbare Gerät aufweist:
- eine Emissionseinheit, insbesondere einen Bildschirm, zur Emission einer ersten elektromagnetischen Strahlung,
- eine Aufnahmeeinheit, insbesondere eine Kamera, zur Aufnahme einer zweiten elektromagnetischen Strahlung und
- zumindest einen Prozessor,
wobei der zumindest eine Prozessor konfiguriert ist zum
- Bewirken einer Beaufschlagung einer bereitgestellten Probe des zu analysierenden Betriebsstoffes mit der ersten elektromagnetischen Strahlung anhand der Emissionseinheit,
- Bewirken einer Aufnahme der zweiten elektromagnetischen Strahlung, welche von der Probe während der Beaufschlagung mit der ersten elektromagnetischen Strahlung ausgesandt wird, anhand der Aufnahmeeinheit,
- Bewirken einer Ermittlung des Zustandes des Betriebsstoffes anhand einer Analyse der zweiten elektromagnetischen Strahlung unter Berücksichtigung der ersten elektromagnetischen Strahlung.

Der zumindest eine Prozessor des tragbaren Gerätes ist dabei ausgelegt, die Ermittlung des Zustandes des Betriebsstoffes anhand einer Analyse der zweiten elektromagnetischen Strahlung unter Berücksichtigung der ersten elektromagnetischen Strahlung zu bewirken. Die Analyse kann dabei insbesondere durch den zumindest einen Prozessor des tragbaren Gerätes selbst durchgeführt werden. Alternativ ist auch denkbar, dass das tragbare Gerät zumindest die aufgenommene zweite elektromagnetische Strahlung an einen datentechnisch verbundenen Rechner übermittelt und der Rechner anhand zumindest der übermittelten zweiten elektromagnetischen Strahlung den Zustand des Betriebsstoffes ermittelt. Die Übermittlung umfasst dabei vorzugsweise Informationen zum jeweiligen Spektrum der elektromagnetischen Strahlung. Bei dieser Alternative übermittelt der Rechner vorzugsweise den ermittelten Zustand an das tragbare Gerät, insbesondere um von dem tragbaren Gerät angezeigt zu werden.

Insbesondere wird das vorgeschlagene tragbare Gerät zur Durchführung zumindest eines der oben erläuterten Verfahren oder zur Durchführung eines der nachfolgend erläuterten vorteilhaften Ausgestaltungen des Verfahrens zur Ermittlung des Zustandes des Betriebsstoffes verwendet. Ferner kann ein System zur Ermittlung des Zustandes des Betriebsstoffes der Maschine vorgesehen sein, wobei das System zumindest das tragbare Gerät und den genannten Rechner umfasst.

Schließlich ergibt sich noch eine Lösung der ersten Aufgabe durch ein nichtflüchtiges computerlesbares Speichermedium, das darin gespeicherte Daten aufweist, die Anweisungen darstellen, die von einem programmierten Prozessor zur Ermittlung eines Zustandes eines Betriebsstoffes einer Maschine ausführbar sind, wobei das Speichermedium Anweisungen aufweist zum
- Bewirken einer Beaufschlagung einer bereitgestellten Probe des zu analysierenden Betriebsstoffes mit einer ersten elektromagnetischen Strahlung,
- Bewirken einer Aufnahme einer zweiten elektromagnetischen Strahlung, welche von der Probe während der Beaufschlagung mit der ersten elektromagnetischen Strahlung ausgesandt wird,
- Bewirken einer Ermittlung des Zustandes des Betriebsstoffes anhand einer Analyse der zweiten elektromagnetischen Strahlung unter Berücksichtigung der ersten elektromagnetischen Strahlung.

Insbesondere wird das vorgeschlagene nichtflüchtige computerlesbare Speichermedium zur Durchführung zumindest eines der oben erläuterten Verfahren oder zur Durchführung eines der nachfolgend erläuterten vorteilhaften Ausgestaltungen des Verfahrens zur Ermittlung des Zustandes des Betriebsstoffes verwendet, wobei die Anweisungen des vorgeschlagenen nichtflüchtigen computerlesbaren Speichermediums vorzugsweise auf dem oben erläuterten tragbaren Gerät gespeichert sind.

Bei einer vorteilhaften Ausgestaltung der Erfindung umfasst das tragbare Gerät ein Smartphone oder einen Tabletcomputer, wobei die Beaufschlagung mit der ersten elektromagnetischen Strahlung anhand einer Emissionseinheit, insbesondere eines Bildschirms, des tragbaren Gerätes erfolgt und wobei die Aufnahme der zweiten elektromagnetischen Strahlung anhand einer Aufnahmeeinheit, insbesondere einer Kamera, des tragbaren Gerätes erfolgt.

Die Verwendung eines derartigen, tragbaren Gerätes stellt eine beträchtliche Erleichterung der Ermittlung des Zustandes des Betriebsstoffes der Maschine dar, da die Zustandsermittlung damit praktisch ohne zusätzliche, anzuschaffende Geräte durchgeführt werden kann. Denn heutzutage sind Smartphones oder Tabletcomputer derart weit verbreitet, dass ohne Weiteres angenommen werden kann, dass das Personal zum Betrieb der Maschine oder das Personal für die Inspektion oder den Service der Maschine stets ein Smartphone oder einen Tabletcomputer bei sich hat. Das jeweilige tragbare Gerät müsste dann nur noch mit einer entsprechenden Anwendung oder App in Form eines Programmes ertüchtigt werden, um das vorgeschlagene Verfahren zur Zustandsermittlung des Betriebsstoffes durchzuführen.

Vorzugsweise ist das tragbare Gerät dabei als Smartphone oder Tabletcomputer ausgestaltet, welches bzw. welcher einen entsprechenden Bildschirm und eine entsprechende Kamera aufweist.

Bei einer weiteren vorteilhaften Ausgestaltung der Erfindung umfasst das tragbare Gerät dabei weiterhin eine Komponente aufweisend die Emissionseinheit und/oder die Aufnahmeeinheit umfasst, wobei die Komponente datentechnisch mit dem Smartphone oder dem Tabletcomputer verbunden ist.

Diese Ausgestaltung des tragbaren Gerätes ist insbesondere dann vorteilhaft, wenn die Möglichkeiten des Smartphones bzw. Tabletcomputers zur Beaufschlagung der Probe mit der ersten elektromagnetischen Strahlung bzw. zur Aufnahme der zweiten elektromagnetischen Strahlung von der Probe nicht ausreichen.

Vorzugsweise ist die Emissionseinheit und/oder die Aufnahmeeinheit der Komponente dazu geeignet, eine Infrarot-Strahlung abzugeben und/oder aufzunehmen, wobei bzgl. der Infrarot-Strahlung auf nachfolgende Erläuterungen verwiesen wird. Die Komponente ist datentechnisch, bspw. kabelgebunden oder kabellos, insbesondere über Bluetooth, WLAN etc., mit dem Smartphone bzw. dem Tabletcomputer verbunden und kann zur Durchführung des vorgeschlagenen Verfahrens verwendet werden.

Bei einer weiteren vorteilhaften Ausgestaltung der Erfindung umfasst die erste elektromagnetische Strahlung und/oder die zweite elektromagnetische Strahlung sichtbares Licht und/oder infrarote Strahlung.

Das sichtbare Licht weist dabei Wellenlängen zwischen etwa 380 nm für violettes Licht und 750 nm für rotes Licht auf, während infrarote Strahlung Wellenlängen im Bereich von 780 nm für nahes Infrarot bis 1 mm für fernes Infrarot aufweist. Schon durch die Bestrahlung der zu untersuchenden Probe mit sichtbarem Licht können oftmals recht gute und zuverlässige Ergebnisse erzielt werden, bspw. durch die oben schon erwähnte Farbe des Betriebsmittels bzw. einer hohen Reflexionsrate aufgrund von Eisen- oder Stahlspänen oder -abrieb.

Wie bspw. aus der Infrarotspektrometrie, insbesondere der Fourier-Transformations-Infrarotspektrometrie bekannt ist, ist dabei besonders jener Teil des Infrarot-Spektrums mit Wellenzahlen zwischen 4000 cm⁻¹ und 900 cm⁻¹ interessant, was einem Wellenlängen-Bereich zwischen 2,5 µm und 11 µm entspricht. Dies liegt daran, dass bspw. bei Maschinenöl in diesem Teil des Spektrums einige Oxidationsprodukte für Wellenzahlen im Bereich von 1750 cm⁻¹ bis 1500 cm⁻¹ nachgewiesen werden können und diese Oxidationsprodukte einen Hinweis auf einen schlechten Zustand des Maschinenöls geben.

Bei einer weiteren vorteilhaften Ausgestaltung der Erfindung wird die Probe während einer ersten Zeitdauer mit zumindest einem ersten Frequenzbereich der ersten elektromagnetischen Strahlung beaufschlagt und die zweite elektromagnetische Strahlung die von der Probe ausgesandt wird, wird aufgenommen, und während einer zweiten Zeitdauer wird die Probe mit zumindest einem zweiten Frequenzbereich der ersten elektromagnetischen Strahlung beaufschlagt und die zweite elektromagnetische Strahlung, die von der Probe ausgesandt wird, wird aufgenommen.

Beispielsweise kann während einer ersten Zeitdauer die Probe rotem Licht mit einer Wellenlänge im Bereich von 630 nm bis 700 nm ausgesetzt werden, um anschließend während einer zweiten Zeitdauer orangenfarbenem Licht mit einer Wellenlänge im Bereich von 590 nm bis 630 nm ausgesetzt zu werden. Zusätzlich bzw. alternativ sind andere Wellenlängen sind denkbar, wie z.B. gelbes Licht mit einer Wellenlänge im Bereich von 560 nm bis 590 nm und/oder grünes Licht mit einer Wellenlänge zwischen 490 nm bis 560 nm und/oder Infrarotstrahlung, insbesondere mit Wellenzahlen im Bereich von 1750 cm⁻¹ bis 1500 cm⁻¹, wie weiter oben schon erläutert wurde.

Die jeweilige Zeitdauer kann dabei vergleichsweise klein sein, so zum Beispiel einige Millisekunden bis einige Sekunden, so dass selbst dann, wenn mehrere Frequenzbänder auf die Probe beaufschlagt werden, die vollständige Untersuchung der Probe innerhalb kurzer Zeit abgeschlossen werden kann.

Durch die sequenzielle Untersuchung der Probe mit Licht verschiedener Wellenlänge, insbesondere monochromatischer bzw. nahezu monochromatischer Strahlung, wird die Analyse der zweiten elektromagnetischen Strahlung weniger komplex und somit erleichtert. Dies liegt insbesondere daran, dass für eine jeweilige, definierte erste elektromagnetische Strahlung eines definierten nahezu monochromatischen Wellenlängenbereiches ein Spektrum einer jeweiligen zweiten elektromagnetischen Strahlung aufgenommen und analysiert werden kann und somit Überlagerungen von jeweiligen zweiten elektromagnetischen Strahlungen vermieden werden, die von unterschiedlichen ersten elektromagnetischen Strahlungen unterschiedlicher Wellenlängenbereiche herrühren.

Bei einer weiteren vorteilhaften Ausgestaltung der Erfindung wird bei der Ermittlung des Zustandes des Betriebsstoffes die Sorte des in der Maschine verwendeten Betriebsstoffes berücksichtigt.

Für das Beispiel der Maschinenöle kann als Sorte dabei insbesondere berücksichtigt werden, ob es sich um ein synthetisches, teilsynthetisches oder mineralisches Öl handelt. Weiterhin können bspw. physikalische oder chemische Charakteristika erfasst werden, wie zum Beispiel die Viskosität, die Dichte, der Anilinpunkt, der Tropfpunkt, der Stockpunkt usw.

Indem die Sorte des in der Maschine verwendeten Betriebsstoffes berücksichtigt wird, kann bspw. die Auswahl der zu beaufschlagenden ersten elektromagnetischen Strahlungsbereiche optimal ausgewählt werden, wozu vorzugsweise entsprechende Listen vorbereitet, hinterlegt und verwendet werden. Insbesondere kann die erhaltene, zweite elektromagnetische Strahlung dank der berücksichtigten Sorte besser interpretiert werden, da bspw. die aufgenommene zweite elektromagnetische Strahlung mit einer zu erwartenden zweiten elektromagnetischen Strahlung verglichen werden kann. Vorzugsweise werden hierzu entsprechende zweite elektromagnetische Strahlungen bzw. Spektren von bekannten Sorten von Betriebsstoffen und bei bekannten Zuständen, bspw. von gut bis schlecht, in entsprechenden Tabellen hinterlegt, um für derartige Vergleiche zur Verfügung zu stehen.

Ein guter Zustand liegt insbesondere vor, wenn der Betriebsstoff noch einwandfrei ist und die Maschine ohne Bedenken und für längere Zeit weiterbetrieben werden kann. Ein schlechter Zustand liegt hingegen bspw. vor, wenn der Betriebsstoff umgehend ersetzt werden sollte, da ein weiterer Betrieb der Maschine mit einer Beeinträchtigung bis hin zu einer Beschädigung der Maschine aufgrund des schlechten Zustandes des Betriebsstoffes einhergeht. Dabei sind auch Zwischenstufen vorstellbar, so dass bspw. bei einem mittleren Zustand davon ausgegangen werden kann, dass der Betriebsstoff innerhalb einiger Wochen ersetzt werden sollte, jedoch die Maschine zunächst weiterbetrieben werden kann, ohne dabei eine Beeinträchtigung oder Beschädigung befürchten zu müssen.

Durch die Berücksichtigung der Sorte des Betriebsstoffes lässt sich die Zuverlässigkeit bei der Ermittlung des Zustandes des Betriebsstoffes somit beträchtlich erhöhen.

Bei einer weiteren vorteilhaften Ausgestaltung der Erfindung wird bei der Ermittlung des Zustandes des Betriebsstoffes ein Vergleich mit einem Referenzwert für die jeweilige zweite elektromagnetische Strahlung vorgenommen.

Beispielsweise kann der jeweilige Referenzwert, wie oben im Zusammenhang mit der Berücksichtigung der Sorte des Betriebsstoffes schon erläutert, eine zu erwartende zweite elektromagnetische Strahlung bzw. ein zu erwartendes Spektrum sein, welches passend für den jeweils vorliegenden, in der Maschine verwendeten Betriebsstoff hinterlegt wurde, um für derartige Vergleiche zur Verfügung zu stehen. Die zu erwartende zweite elektromagnetische Strahlung bzw. das zu erwartende Spektrum wird dabei vorzugsweise für unterschiedliche mögliche Zustände, insbesondere von gut bis schlecht, hinterlegt.

Insbesondere kann der Referenzwert die jeweilige zweite elektromagnetische Strahlung einer früheren Ermittlung des Zustandes des gleichen Betriebsstoffes umfassen bzw. sein, welcher einen bekannten Zustand, insbesondere einen guten oder einen schlechten Zustand, aufweist. So wird bspw. für einen bekannten, im Einsatz befindlichen Betriebsstoff jeweils eine passende zweite elektromagnetische Strahlung für die denkbaren Zustände, also insbesondere von gut bis schlecht, aufgenommen und hinterlegt. Die dazu erforderlichen Analysetätigkeiten können bspw. in professionell ausgerüsteten Analyselabors durchgeführt werden, welche hierzu den gleichen Betriebsstoff verwenden und die Ergebnisse anschließend zur Verfügung stellen und bspw. in hinterlegten Tabellen verfügbar machen.

Ist für einen bekannten, im Einsatz befindlichen Betriebsstoff dabei eine besondere Belichtung mit einer ausgewählten ersten elektromagnetischen Strahlung erforderlich, kann auch diese spezielle erste elektromagnetische Strahlung hinterlegt werden.

Die Hinterlegung der erwähnten Daten kann dabei bspw. in Tabellen erfolgen, auf welche im Rahmen des vorgeschlagenen Verfahrens zurückgegriffen wird.

Insbesondere kann der Referenzwert die jeweilige zweite elektromagnetische Strahlung einer früheren Ermittlung des Zustandes desselben Betriebsstoffes umfassen bzw. sein, wobei die frühere Ermittlung insbesondere während bzw. vor der Befüllung der Maschine mit diesem Betriebsstoff vorgenommen wurde. Somit wird ein und dasselbe Betriebsstoff zur Ermittlung des Referenzwertes und anschließend zur Ermittlung des Zustandes verwendet. Der direkt vor der Befüllung der Maschine ermittelte Referenzwert der Betriebsstoffes wird mit dem früher ermittelten allgemeinen Referenzwert für diesen Betriebsstoff abgeglichen. Dadurch lassen sich Ungenauigkeiten bspw. aufgrund von zulässigen Schwankungsbreiten bei den Eigenschaften des Betriebsstoffes vermeiden, wodurch die Präzision bei der Ermittlung des Zustandes des Betriebsstoffes erhöht wird.

Zusammengefasst lässt sich durch den Vergleich mit einem Referenzwert für die jeweilige zweite elektromagnetische Strahlung somit die Zuverlässigkeit bei der Ermittlung des Zustandes des Betriebsstoffes beträchtlich erhöhen.

Bei einer weiteren vorteilhaften Ausgestaltung der Erfindung umfasst die Ermittlung des Zustandes des Betriebsstoffes eine quantitative Analyse von zumindest einem zu analysierenden Stoff.

Für das obige Beispiel des Maschinenöls kann der jeweilige, zu analysieren der Stoff bspw. Wasser oder Eisen- oder Stahlspäne oder -abrieb sein. Die quantitative Analyse zeigt dabei insbesondere auf, wie viel des zu analysierenden Stoffes in dem Betriebsstoff vorhanden ist. Dadurch lassen sich besonders genaue Aussagen über den Zustand des Betriebsstoffes treffen.

Bei einer weiteren vorteilhaften Ausgestaltung der Erfindung erfolgt die Analyse der zweiten elektromagnetischen Strahlung unter Berücksichtigung der ersten elektromagnetischen Strahlung durch das tragbare Gerät.

Indem das tragbare Gerät selbst die genannte Analyse durchführt, kann die Ermittlung des Zustandes des Betriebsstoffes zum einen direkt vor Ort bei der Untersuchung der Probe und zum anderen innerhalb kurzer Zeit, bspw. innerhalb weniger Minuten oder innerhalb von bis zu zwei Stunden, erfolgen. Ist das tragbare Gerät mit entsprechenden Algorithmen und/oder Vergleichsdaten ausgestattet, insbesondere den oben erläuterten Referenzwerten bzw. Vergleichsdaten bezüglich der Sorte des Betriebsstoffes, so kann die genannte Ermittlung sogar ohne eine datentechnische Verbindungen, insbesondere mobile Internetverbindung oder Mobilfunkverbindung erfolgen, was vor allem bei entlegenen Einsatzorten der Maschine von großem Vorteil sein kann.

Dies ermöglicht es dem Bedienpersonal des tragbaren Gerätes, insbesondere dem Maschinenbetreiber direkte Rückmeldung zum Zustand des Betriebsstoffes zu geben und ggf. Vorschläge zum weiteren Vorgehen zu unterbreiten.

Bei einer alternativen, weiteren vorteilhaften Ausgestaltung der Erfindung übermittelt das tragbare Gerät zumindest die aufgenommene zweite elektromagnetische Strahlung an einen datentechnisch verbundenen Rechner, wobei der Rechner anhand zumindest der übermittelten zweiten elektromagnetischen Strahlung den Zustand des Betriebsstoffes ermittelt.

Der Rechner kann bspw. von einem Service-Dienstleister, dem Maschinenhersteller oder -verkäufer oder dem Hersteller des Betriebsstoffes betrieben werden, um das vorgeschlagene Verfahren zu unterstützen bzw. als Dienstleistung anzubieten. In dem genannten Rechner können bspw. umfangreiche Datenbanken angelegt bzw. vorhanden sein, in welchen bspw. detaillierte Vergleichsdaten und/oder ausgeklügelte Analysealgorithmen abgelegt sind. Der genannte Rechner weist dabei vorzugsweise eine vergleichsweise große Rechenleistung auf, insbesondere im Vergleich zu dem tragbaren Gerät. Bspw. kann der Rechner bzw. seine Datenbank durch aktuelle Vorgänge zur Ermittlung des Zustandes von Betriebsstoffen ergänzt, erweitert bzw. aktualisiert werden. Insbesondere können damit Quervergleiche mit ähnlichen Maschinen und/oder Betriebsstoffen vorgenommen werden, um verfeinerte Aussagen zum Zustand des Betriebsstoffes zu treffen.

Hierzu sind der Rechner und das tragbare Gerät datentechnisch verbunden, bspw. über eine insbesondere mobile Internetverbindung bzw. Mobilfunkverbindung.

Insbesondere kann vorgesehen sein, dass das tragbare Gerät zusätzlich die erste elektromagnetische Strahlung und/oder weitere Informationen, wie zum Beispiel die Sorte des Betriebsstoffes, an einen datentechnisch verbundenen Rechner übermittelt, wobei der Rechner diese zusätzlichen Daten vorzugsweise bei der genannten Analyse berücksichtigt. Die Übermittlung umfasst dabei vorzugsweise Informationen zum jeweiligen Spektrum der elektromagnetischen Strahlung.

Bei einer weiteren vorteilhaften Ausgestaltung der Erfindung übermittelt der Rechner dabei den ermittelten Zustand an das tragbare Gerät.

Durch die Übermittlung des ermittelten Zustandes an das tragbare Gerät wird dem Bedienpersonal des tragbaren Gerätes ermöglicht, insbesondere dem Maschinenbetreiber direkte und schnelle Rückmeldung zum Zustand des Betriebsstoffes zu geben und ggf. Vorschläge zum weiteren Vorgehen zu unterbreiten.

Vorzugsweise sind der Rechner und das tragbare Gerät hierzu datentechnisch verbunden, bspw. über eine insbesondere mobile Internetverbindung bzw. Mobilfunkverbindung.

Bei einer weiteren vorteilhaften Ausgestaltung der Erfindung zeigt das tragbare Gerät den ermittelten Zustand des Betriebsstoffes an.

Durch die Anzeige des ermittelten Zustandes auf dem tragbaren Gerät erhält das Bedienpersonal des tragbaren Gerätes die unmittelbare Rückmeldung bezüglich der vorgenommenen Ermittlung des Zustandes des Betriebsstoffes. Damit wird das Bedienpersonal des tragbaren Gerätes in die Lage versetzt, insbesondere dem Maschinenbetreiber direkte und schnelle Rückmeldung zum Zustand des Betriebsstoffes zu geben und ggf. Vorschläge zum weiteren Vorgehen zu unterbreiten. Die Anzeige kann insbesondere durch die Emissionseinheit bzw. den Bildschirm des oben erläuterten Smartphones bzw. Tabletcomputers erfolgen.

Insbesondere sehen die vorgeschlagenen Verfahren und Vorrichtungen folgende Aspekte vor. Es wird die Erzeugung von Emissionsspektren in definierten, unterschiedlichen Frequenzbereichen mit Hilfe des Bildschirms eines Smartphones oder Tabletcomputers durch eine Bestrahlung des Betriebsstoffes, insbesondere Öls, mit unterschiedlichen Farben und damit mit Licht verschiedener Frequenzbänder vorgenommen. Eine Aufnahme der Emissionsspektren der verschiedenen Frequenzbänder über einen definierten Zeitbereich erfolgt mit der Smartphone- bzw. Tabletcomputer-Frontkamera. Dabei kann eine Angabe der eingesetzten Ölsorte bzw. Sorte des Betriebsstoffes in der Smartphone- bzw. Tabletcomputer-App erfolgen. Eine automatisierte Auswertung der Spektren kann bspw. im Smartphone bzw. Tabletcomputer oder offline, insbesondere nach elektronischem Versenden der Spektren zu einem Dienstleister, mit Hilfe eines Auswertealgorithmus erfolgen.

Beispielsweise kann dabei eine qualitative Analyse der Spektren vorgenommen werden, wozu eine Fourier-Transformation der Spektren durchgeführt wird. Weiterhin wird bspw. ein Vergleich der Spektren mit dem Referenzspektrum des Öls bzw. Betriebsstoffes zum Zeitpunkt der Befüllung der Arbeitsmaschine und mit dem Referenzspektrum des Behälters, aufgenommen direkt vor Aufnahme der Ölprobe - soweit zur Analyse erforderlich - vorgenommen. Zusätzlich oder alternativ können die aufgenommenen Spektren mit Referenzspektren der gleichen Ölsorte bzw. Sorte des Betriebsstoffes verglichen werden, insbesondere mit Spektren von gutem, verwendbarem Öl bzw. Betriebsstoff und auch mit Spektren von schlechtem, verbrauchtem Öl bzw. Betriebsstoff. Ferner kann eine Rückmeldung des Ergebnisses im Smartphone bzw. Tabletcomputer, insbesondere in der Smartphone- bzw. Tabletcomputer-App oder per elektronischem Versand erfolgen, d.h. insbesondere die Nachricht, ob das Öl bzw. der Betriebsstoff gut oder schlecht ist.

Zusätzlich kann eine quantitative Analyse der Spektren erfolgen, indem zusätzlich die aufgenommenen Spektren mit Referenzspektren der gleichen Ölsorte bzw. der gleichen Sorte des Betriebsstoffes verglichen werden, bei denen die absolute Menge der zu analysierenden Inhaltsstoffe bekannt ist. Zusätzlich oder alternativ kann ein Vergleich mit Referenzspektren der Inhaltsstoffe, bei denen die absolute Menge der Inhaltsstoffe bekannt ist, erfolgen. Dadurch ist die Bestimmung der absoluten Mengen der zu analysierenden Inhaltsstoffe möglich. Ferner kann eine Rückmeldung des Ergebnisses im Smartphone bzw. Tabletcomputer, insbesondere in der Smartphone- bzw. Tabletcomputer-App oder per elektronischem Versand erfolgen, d.h. insbesondere die Nachricht, ob das Öl bzw. der Betriebsstoff gut oder schlecht ist und welche absolute Menge eines bestimmten Inhaltsstoffes in dem Öl bzw. Betriebsstoff gefunden wurde.

Im Folgenden wird die Erfindung anhand der in den Figuren dargestellten Ausführungsbeispiele näher beschrieben und erläutert. Es zeigen:
- FIG 1: ein beispielhaftes Ablaufschema des vorgeschlagenen Verfahrens zur Ermittlung des Zustandes eines Betriebsstoffes,
- FIGS 2-4: ein erstes bis drittes Ausführungsbeispiel des vorgeschlagenen, tragbaren Gerätes,
- FIG 5: einen beispielhaften zeitlichen Ablauf der Beaufschlagung der Probe mit der ersten elektromagnetischen Strahlung, und
- FIG 6: ein viertes Ausführungsbeispiel des vorgeschlagenen, tragbaren Gerätes.

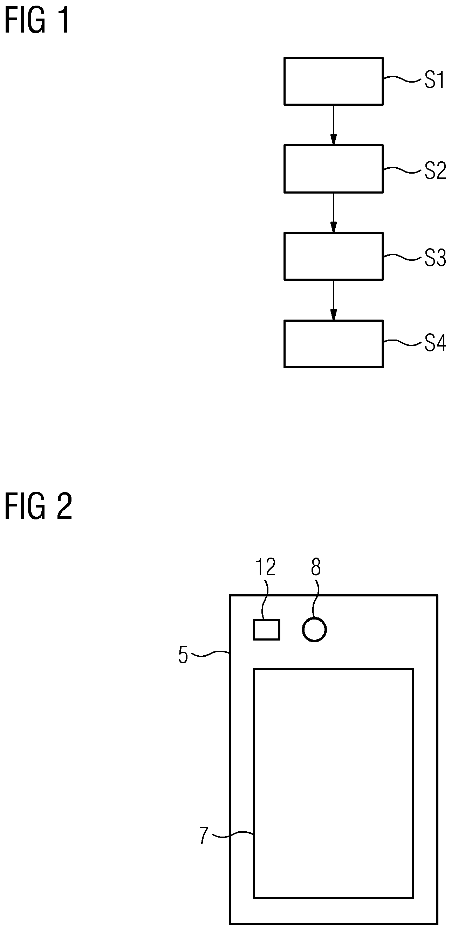

Figur 1 zeigt ein beispielhaftes Ablaufschema des vorgeschlagenen Verfahrens zur Ermittlung des Zustandes eines Betriebsstoffes 1 einer Maschine 2. Gleiche Bezugszeichen wie in den übrigen Figuren bezeichnen dabei gleiche Gegenstände.

In einem Schritt S1 wird eine Probe 3 des zu analysierenden Betriebsstoffes 1 bereitgestellt. In einem zweiten Schritt S2 wird die bereitgestellte Probe 3 mit einer ersten elektromagnetischen Strahlung 4 mittels eines tragbaren Gerätes 5 beaufschlagt. In einem dritten Schritt S3 wird eine zweite elektromagnetische Strahlung 6, welche von der Probe 3 während der Beaufschlagung mit der ersten elektromagnetischen Strahlung 4 ausgesandt wird, mittels des tragbaren Gerätes 5 aufgenommen. In einem vierten Schritt wird der Zustand des Betriebsstoffes 1 anhand einer Analyse der zweiten elektromagnetischen Strahlung 6 unter Berücksichtigung der ersten elektromagnetischen Strahlung 4 ermittelt.

Vorzugsweise umfasst das tragbare Gerät 5 ein Smartphone oder einen Tabletcomputer bzw. ist als solches ausgestaltet. Die erste elektromagnetische Strahlung 4 und/oder die zweite elektromagnetische Strahlung 6 kann sichtbares Licht und/oder Infrarotstrahlung umfassen. Vorzugsweise wird die Probe 3 während einer ersten Zeitdauer 9 mit zumindest einem ersten Frequenzbereich 10 der ersten elektromagnetischen Strahlung 4 und während einer zweiten Zeitdauer 9' zumindest einem zweiten Frequenzbereich 10' der ersten elektromagnetischen Strahlung 4 beaufschlagt.

Bei einem zusätzlichen Schritt kann vorgesehen sein, dass die Analyse der zweiten elektromagnetischen Strahlung 6 unter Berücksichtigung der ersten elektromagnetischen Strahlung 4 durch das tragbare Gerät 5 erfolgt. Alternativ kann ein zusätzlicher Schritt vorgesehen sein, welcher vorsieht, dass das tragbare Gerät 5 zumindest die aufgenommene zweite elektromagnetische Strahlung 6 an einen datentechnisch verbundenen Rechner 11 übermittelt und der Rechner 11 anhand der übermittelten zweiten elektromagnetischen Strahlung 6 den Zustand des Betriebsstoffes 1 ermittelt. Für diesen alternativen Schritt kann anschließend ein weiterer Schritt vorgesehen sein, gemäß welchem der Rechner 11 den ermittelten Zustand an das tragbare Gerät 5 übermittelt.

Vorzugsweise wird ferner ein Schritt vorgesehen, bei welchem das tragbare Gerät 5 den ermittelten Zustand des Betriebsstoffes 1 anzeigt.

Insbesondere können die erläuterten Schritte als Teil eines Serviceverfahrens für die Maschine 2 verwendet werden, bei deren Betrieb der Betriebsstoff 1 verwendet wird und welches, für den Fall dass der ermittelte Zustand des Betriebsstoffes 1 unzureichend ist, zusätzlich den Schritt aufweist, dass eine elektronische Bestellung von neuem Betriebsstoff 1 vorgenommen wird, um den bisher verwendeten Betriebsstoff 1 mit dem unzureichenden Zustand zu ersetzen, und/oder das eine elektronische Bestellung eines Servicetermins vorgenommen wird, um den bisher verwendeten Betriebsstoff 1 mit dem unzureichenden Zustand zu ersetzen.

Figur 2 zeigt ein erstes Ausführungsbeispiel des vorgeschlagenen, tragbaren Gerätes 5.

Das tragbare Gerät 5 dient zur Ermittlung eines Zustandes eines Betriebsstoffes 1 einer Maschine 2 und weist eine Emissionseinheit 7, eine Aufnahmeeinheit 8 und zumindest einen Prozessor 12 auf. Die Emissionseinheit 7 ist insbesondere als Bildschirm ausgestaltet und ist für die Emission einer ersten elektromagnetischen Strahlung 4 ausgelegt. Die Aufnahmeeinheit ist insbesondere als Kamera ausgestaltet und ist für die Aufnahme einer zweiten elektromagnetischen Strahlung 6 ausgelegt. Der zumindest eine Prozessor 12 ist konfiguriert zum Bewirken einer Beaufschlagung einer bereitgestellten Probe 3 des zu analysierenden Betriebsstoffes 1 mit der ersten elektromagnetischen Strahlung 4 anhand der Emissionseinheit 7. Weiterhin ist der zumindest eine Prozessor 12 konfiguriert zum Bewirken einer Aufnahme der zweiten elektromagnetischen Strahlung 6, welche von der Probe 3 während der Beaufschlagung mit der ersten elektromagnetischen Strahlung 4 ausgesandt wird, anhand der Aufnahmeeinheit 8. Ferner ist der zumindest eine Prozessor 12 konfiguriert zum Bewirken einer Ermittlung des Zustandes des Betriebsstoffes 1 anhand einer Analyse der zweiten elektromagnetischen Strahlung 6 unter Berücksichtigung der ersten elektromagnetischen Strahlung 4.

Insbesondere umfasst das tragbare Gerät 5 ein Smartphone oder einen Tabletcomputer bzw. ist als solches ausgestaltet. Dabei können ein Bildschirm des Smartphones oder des Tabletcomputers als Emissionseinheit 7 und eine Kamera des Smartphones oder des Tabletcomputers als Aufnahmeeinheit 8 dienen.

Vorzugsweise zeigt das tragbare Gerät 5 den ermittelten Zustand des Betriebsstoffes 1 an.

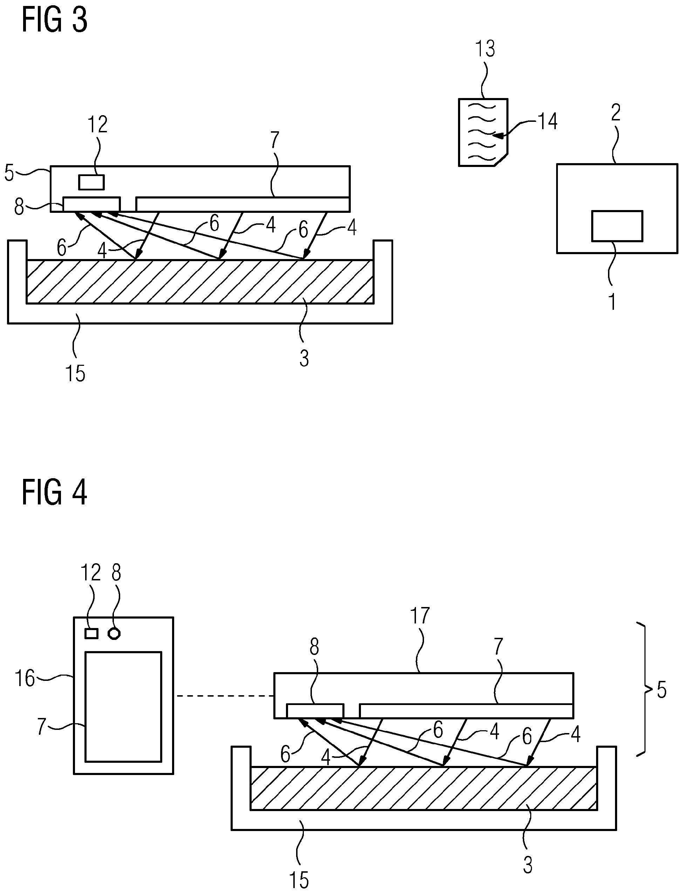

Figur 3 zeigt ein zweites Ausführungsbeispiel des vorgeschlagenen, tragbaren Gerätes 5. Dargestellt wird dabei ein beispielhafter Einsatz des tragbaren Gerätes 5 zur Ermittlung eines Zustandes eines Betriebsstoffes 1 einer Maschine 2, bspw. im Rahmen des in Figur 1 dargestellten und erläuterten beispielhaften Ablaufschemas. Das tragbare Gerät 5 des zweiten Ausführungsbeispiels weist dabei einige Ähnlichkeiten zum ersten Ausführungsbeispiel auf.

Wie in Figur 3 dargestellt ist, ist eine Probe 3 des zu analysierenden Betriebsstoffes 1 bereitgestellt, wozu im Rahmen des Ausführungsbeispiels ein Behälter 15 zum Einsatz kommt. Die bereitgestellte Probe 3 wird mittels des tragbaren Gerätes 5, insbesondere mittels der Emissionseinheit 7, mit einer ersten elektromagnetischen Strahlung 4 beaufschlagt. Das tragbare Gerät 5, insbesondere die Aufnahmeeinheit 8, nimmt eine zweite elektromagnetische Strahlung 6 auf, welche von der Probe 3 während der Beaufschlagung mit der ersten elektromagnetischen Strahlung 4 ausgesandt wird. Der Zustand des Betriebsstoffes 1 wird, vorzugsweise durch das tragbare Gerät 5, anhand einer Analyse der zweiten elektromagnetischen Strahlung 6 unter Berücksichtigung der ersten elektromagnetischen Strahlung 4 ermittelt.

Die Ausführung der erläuterten Beaufschlagung, Aufnahme und Ermittlung wird dabei von dem Prozessor 12 des tragbaren Gerätes 5 bewirkt, wozu der Prozessor 12 entsprechend konfiguriert ist. Hierzu kommt ein nichtflüchtiges computerlesbares Speichermedium 13 zum Einsatz, das darin gespeicherte Daten 14 aufweist, die Anweisungen darstellen, die von dem entsprechend programmierten Prozessor 12 zur Ermittlung des Zustandes des Betriebsstoffes 1 der Maschine 2 ausführbar sind.

Vorzugsweise ist das tragbare Gerät 5, insbesondere das Speichermedium 13, mit entsprechenden Algorithmen und/oder Vergleichsdaten ausgestattet, insbesondere den oben erläuterten Referenzwerten bzw. Vergleichsdaten bezüglich der Sorte des Betriebsstoffes 1, um eine schnelle und zuverlässige Ermittlung des Zustandes des Betriebsstoffes 1 zu gewährleisten.

Vorzugsweise zeigt das tragbare Gerät 5 den ermittelten Zustand des Betriebsstoffes 1 an, bspw. mittels der Emissionseinheit 7.

Figur 4 zeigt ein drittes Ausführungsbeispiel des vorgeschlagenen, tragbaren Gerätes 5. Da das dritte Ausführungsbeispiel einige Ähnlichkeiten mit dem zweiten Ausführungsbeispiel aufweist, werden im Folgenden einige Unterschiede erläutert.

Das tragbare Gerät 5 weist neben einem Smartphone 16 oder einem Tabletcomputer 16 weiterhin eine Komponente 17 auf, welche die Emissionseinheit 7 und die Aufnahmeeinheit 8 umfasst. Dabei ist die Komponente 17 datentechnisch mit dem Smartphone 16 oder dem Tabletcomputer 16 verbunden, insbesondere kabelgebunden oder kabellos, insbesondere über Bluetooth, WLAN etc. Auch das Smartphone 16 oder der Tabletcomputer 16 kann dabei eine Emissionseinheit 7 und eine Aufnahmeeinheit 8 aufweisen, diese sind jedoch im Rahmen dieses Ausführungsbeispiels als optional anzusehen.

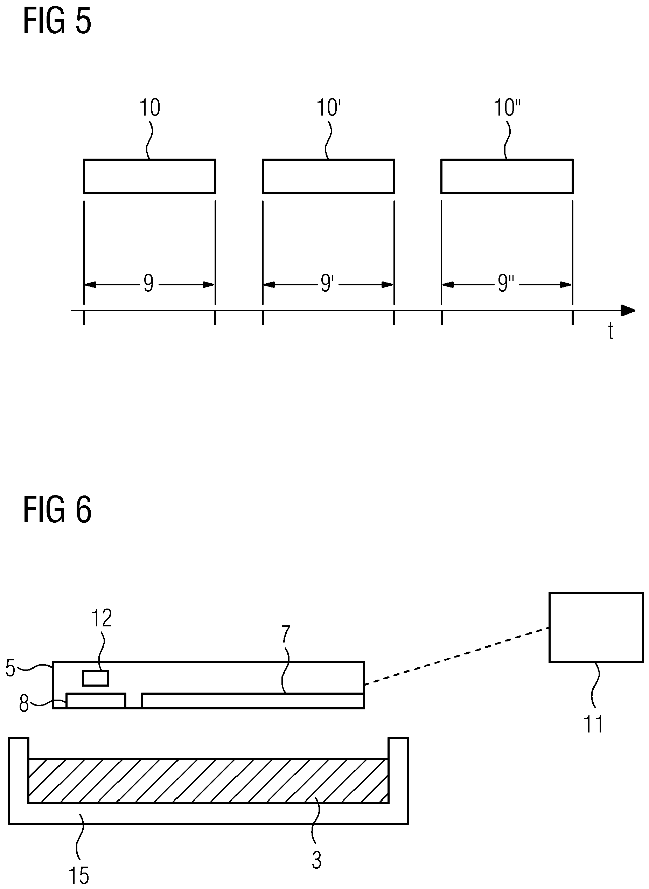

Figur 5 zeigt einen beispielhaften zeitlichen Ablauf der Beaufschlagung der Probe 3 mit der ersten elektromagnetischen Strahlung 4.

Die Probe 3 wird während einer ersten Zeitdauer 9 mit einem ersten Frequenzbereich 10 der ersten elektromagnetischen Strahlung 4 beaufschlagt, bspw. mit rotem Licht mit einer Wellenlänge im Bereich von 630 nm bis 700 nm. Anschließend wird die Probe 3 während einer zweiten Zeitdauer 9' mit einem zweiten Frequenzbereich 10' der ersten elektromagnetischen Strahlung 4 beaufschlagt, bspw. orangenfarbenem Licht mit einer Wellenlänge im Bereich von 590 nm bis 630 nm. Während eines anschließenden dritten Zeitbereichs 9" wird die Probe 3 mit einem dritten Frequenzbereich 10" der ersten elektromagnetischen Strahlung 4 beaufschlagt, bspw. gelbes Licht mit einer Wellenlänge im Bereich von 560 nm bis 590 nm und/oder grünes Licht mit einer Wellenlänge zwischen 490 nm bis 560 nm und/oder Infrarotstrahlung, insbesondere mit Wellenzahlen im Bereich von 1750 cm⁻¹ bis 1500 cm⁻¹.

Figur 6 zeigt ein viertes Ausführungsbeispiel des vorgeschlagenen, tragbaren Gerätes 5. Da das vierte Ausführungsbeispiel einige Ähnlichkeiten mit dem zweiten oder dritten Ausführungsbeispiel aufweist, werden im Folgenden einige Unterschiede erläutert.

Im Rahmen des vierten Ausführungsbeispiels erfolgt die Analyse der zweiten elektromagnetischen Strahlung 6 unter Berücksichtigung der ersten elektromagnetischen Strahlung 4 anhand eines Rechners 11, mit welchem das tragbare Gerät 5 datentechnisch verbunden ist. Hierzu überträgt das tragbare Gerät 5 zumindest die aufgenommene zweite elektromagnetische Strahlung 6 an den Rechner 11. Zusätzlich kann das tragbare Gerät bspw. die erste elektromagnetische Strahlung 4 und/oder weitere Informationen, wie zum Beispiel die Sorte des Betriebsstoffes, an den Rechner 11 übermitteln.

Der Rechner 11 ermittelt insbesondere anhand der vom tragbaren Gerät 5 erhaltenen Daten den Zustand des Betriebsstoffes 1. Vorzugsweise übermittelt der Rechner 11 den ermittelten Zustand an das tragbare Gerät 5.

Vorzugsweise zeigt das tragbare Gerät 5 den ermittelten Zustand des Betriebsstoffes 1 an.

Das tragbare Gerät 5 und der Rechner 11 können bspw. als Teil eines Systems zur Ermittlung des Zustandes des Betriebsstoffes 1 der Maschine 2 ausgebildet sein.

## Patentansprüche

1. Verfahren zur Ermittlung eines Zustandes eines Betriebsstoffes (1) einer Maschine (2) aufweisend die Verfahrensschritte:
- Bereitstellen einer Probe (3) des zu analysierenden Betriebsstoffes (1),
- Beaufschlagen der bereitgestellten Probe (3) mit einer ersten elektromagnetischen Strahlung (4) mittels eines tragbaren Gerätes (5),
- Aufnehmen einer zweiten elektromagnetischen Strahlung (6), welche von der Probe (3) während der Beaufschlagung mit der ersten elektromagnetischen Strahlung (4) ausgesandt wird, mittels des tragbaren Gerätes (5),
- Ermitteln des Zustandes des Betriebsstoffes (1) anhand einer Analyse der zweiten elektromagnetischen Strahlung (6) unter Berücksichtigung der ersten elektromagnetischen Strahlung (4).

2. Verfahren nach Anspruch 1,
wobei das tragbare Gerät (5) ein Smartphone oder einen Tabletcomputer umfasst,
wobei die Beaufschlagung mit der ersten elektromagnetischen Strahlung (4) anhand einer Emissionseinheit (7), insbesondere eines Bildschirms, des tragbaren Gerätes (5) erfolgt und wobei die Aufnahme der zweiten elektromagnetischen Strahlung (6) anhand einer Aufnahmeeinheit (8), insbesondere einer Kamera, des tragbaren Gerätes (5) erfolgt.

3. Verfahren nach Anspruch 2,
wobei das tragbare Gerät (5) weiterhin eine Komponente (17) aufweisend die Emissionseinheit (7) und/oder die Aufnahmeeinheit (8) umfasst und
wobei die Komponente (17) datentechnisch mit dem Smartphone (16) oder dem Tabletcomputer (16) verbunden ist.

4. Verfahren nach einem der vorhergehenden Ansprüche,
wobei die erste elektromagnetische Strahlung (4) und/oder die zweite elektromagnetische Strahlung (6) sichtbares Licht und/oder infrarote Strahlung umfasst.

5. Verfahren nach einem der vorhergehenden Ansprüche,
wobei die Probe (3) während einer ersten Zeitdauer (9) mit zumindest einem ersten Frequenzbereich (10) der ersten elektromagnetischen Strahlung (4) und während einer zweiten Zeitdauer (9') mit zumindest einem zweiten Frequenzbereich (10') der ersten elektromagnetischen Strahlung (4) beaufschlagt wird.

6. Verfahren nach einem der vorhergehenden Ansprüche,
wobei bei der Ermittlung des Zustandes des Betriebsstoffes (1) die Sorte des in der Maschine (2) verwendeten Betriebsstoffes (1) berücksichtigt wird.

7. Verfahren nach einem der vorhergehenden Ansprüche,
wobei bei der Ermittlung des Zustandes des Betriebsstoffes (1) ein Vergleich mit einem Referenzwert für die jeweilige zweite elektromagnetische Strahlung vorgenommen wird.

8. Verfahren nach einem der vorhergehenden Ansprüche,
wobei die Ermittlung des Zustandes des Betriebsstoffes (1) eine quantitative Analyse von zumindest einem zu analysierenden Stoff umfasst.

9. Verfahren nach einem der vorhergehenden Ansprüche,
wobei die Analyse der zweiten elektromagnetischen Strahlung (6) unter Berücksichtigung der ersten elektromagnetischen Strahlung (4) durch das tragbare Gerät (5) erfolgt.

10. Verfahren nach einem der Ansprüche 1 bis 8,
wobei das tragbare Gerät (5) zumindest die aufgenommene zweite elektromagnetische Strahlung (6) an einen datentechnisch verbundenen Rechner (11) übermittelt und
wobei der Rechner (11) anhand zumindest der übermittelten zweiten elektromagnetischen Strahlung (6) den Zustand des Betriebsstoffes (1) ermittelt.

11. Verfahren nach Anspruch 10,
wobei der Rechner (11) den ermittelten Zustand an das tragbare Gerät (5) übermittelt.

12. Verfahren nach einem der vorhergehenden Ansprüche,
wobei das tragbare Gerät (5) den ermittelten Zustand des Betriebsstoffes (1) anzeigt.

13. Serviceverfahren für eine Maschine (2), bei deren Betrieb ein Betriebsstoff (1) verwendet wird, aufweisend die Verfahrensschritte:
- Ermitteln des Zustandes des Betriebsstoffes (1) der Maschine (2) nach einem Verfahren gemäß einem der vorhergehenden Ansprüche,
- falls der ermittelte Zustand des Betriebsstoffes (1) unzureichend ist:
Vornehmen einer elektronischen Bestellung von neuem Betriebsstoff (1), um den bisher verwendeten Betriebsstoff (1) mit dem unzureichenden Zustand zu ersetzen, und/oder Vornehmen einer elektronischen Bestellung eines Servicetermins, um den bisher verwendeten Betriebsstoff (1) mit dem unzureichenden Zustand zu ersetzen.

14. Tragbares Gerät (5) zur Ermittlung eines Zustandes eines Betriebsstoffes (1) einer Maschine (2), wobei das tragbare Gerät (5) aufweist:
- eine Emissionseinheit (7), insbesondere einen Bildschirm, zur Emission einer ersten elektromagnetischen Strahlung (4),
- eine Aufnahmeeinheit (8), insbesondere eine Kamera, zur Aufnahme einer zweiten elektromagnetischen Strahlung (6) und
- zumindest einen Prozessor (12),
wobei der zumindest eine Prozessor (12) konfiguriert ist zum
- Bewirken einer Beaufschlagung einer bereitgestellten Probe (3) des zu analysierenden Betriebsstoffes (1) mit der ersten elektromagnetischen Strahlung (4) anhand der Emissionseinheit (7),
- Bewirken einer Aufnahme der zweiten elektromagnetischen Strahlung (6), welche von der Probe (3) während der Beaufschlagung mit der ersten elektromagnetischen Strahlung (4) ausgesandt wird, anhand der Aufnahmeeinheit (8),
- Bewirken einer Ermittlung des Zustandes des Betriebsstoffes (1) anhand einer Analyse der zweiten elektromagnetischen Strahlung (6) unter Berücksichtigung der ersten elektromagnetischen Strahlung (4).

15. Nichtflüchtiges computerlesbares Speichermedium (13), das darin gespeicherte Daten (14) aufweist, die Anweisungen darstellen, die von einem programmierten Prozessor (12) zur Ermittlung eines Zustandes eines Betriebsstoffes (1) einer Maschine (2) ausführbar sind, wobei das Speichermedium (13) Anweisungen aufweist zum
- Bewirken einer Beaufschlagung einer bereitgestellten Probe (3) des zu analysierenden Betriebsstoffes (1) mit einer ersten elektromagnetischen Strahlung (3),
- Bewirken einer Aufnahme einer zweiten elektromagnetischen Strahlung (6), welche von der Probe (3) während der Beaufschlagung mit der ersten elektromagnetischen Strahlung (4) ausgesandt wird,
- Bewirken einer Ermittlung des Zustandes des Betriebsstoffes (1) anhand einer Analyse der zweiten elektromagnetischen Strahlung (6) unter Berücksichtigung der ersten elektromagnetischen Strahlung (4).
